# EUROPEAN PATENT APPLICATION

(11) **EP 4 148 079 A1**
(43) Date of publication of application: **15.03.2023**
(21) Application number: 21195557.0
(22) Date of filing: 08.09.2021
(51) Int. Cl.: C08G 63/06, A61K 9/28, C08G 63/60, C08G 63/66, C08G 63/78, C09D 167/00, C09D 167/04

(54) **PROCESS FOR PREPARING A SUBSTRATE COMPRISING AN EDIBLE SURFACE COATING**

(71) Applicant: Evonik Operations GmbH, 45128 Essen (DE)
(72) Inventor: The designation of the inventor has not yet been filed
(74) Representative: Evonik Patent Association

(57) **Abstract**

The present invention relates to a process for preparing a substrate comprising an edible surface coating, characterized in that a) a polymer is prepared by reacting at least aleuritic acid as monomer at a temperature of above 100 °C, preferably of from 120 °C to 150 °C while removing water from the reaction mixture, and b) the resulting polymer is applied to the substrate to give an edible surface coating.

## Description

The present invention relates to a process for preparing a substrate comprising an edible surface coating, characterized in that a) a polymer is prepared by reacting at least aleuritic acid as monomer at a temperature of above 100 °C while removing water from the reaction mixture, and b) the resulting polymer is applied to the substrate, to give an edible surface coating.

### STATE OF THE ART

Shellac, a wax-containing resin secreted by the Lac insect Kerria lacca, is known as E904 and is used frequently as glazing agent or edible surface coating. It is used in the food industry, confectionery industry, pharmaceutical industry and other industries, for example wood treatment industry, textiles & leather industry, or as binding agents or varnish, polish or non-edible coatings.

Upon mild hydrolysis shellac gives a complex mix of aliphatic and alicyclic hydroxy acids and their polymers that varies in exact composition depending upon the source of the shellac and the season of collection. The major component of the aliphatic component is aleuritic acid, whereas the main alicyclic component is shellolic acid (Source: en.wikipedia.org, Wikidata item ID: Q429659).

Since shellac is a natural product the composition, transparency, and other properties depend from the source of the natural product. Therefore, properties and composition of shellac varies in a wide range. To come to a product that is commercially applicable several purification (extraction) steps have to be performed.

EP 3 009 464 A1 discloses a process of polymerizing methyl aleuriteate.

GB 1 052 013 A discloses a process for preparing a polymer comprising the step of reacting aleuritic acid and trimellitic anhydride at a temperature of 180 °C. The resulting polymer is used in coating compositions comprising an aminoplast.

Veld Martijn A. J. et al. reported in Journal of Polymer Science Part A: Polymer Chemistry, vol. 45, no. 24, December 15, 2007, pages 5968-5978 a process of polymerizing isopropyl aleuriteate.

Jose Jesus Benítez et al. reported in Journal of Applied Polymer Science (https://doi.org/10.1002/app.41328, first published August 20, 2014) of Polyester films obtained by noncatalyzed melt-condensation polymerization of aleuritic (9,10,16-trihydroxyhexadecanoic) acid in air. The esterification reaction leads to an amorphous rubbery, infusible, and insoluble material, not useable for film forming.

The problem to be solved by the present invention, was to provide polymers usable as glazing agents or edible, preferably edible, surface coatings that have defined properties and a defined composition.

### DISCLOSURE OF THE INVENTION

Surprisingly it has been found that this problem can be solved by the process according to the claims.

Therefore, the objectives are achieved by the invention by a process for preparing a substrate comprising an edible surface coating, wherein a) a polymer is prepared by reacting at least aleuritic acid as monomer at a temperature of above 100 °C while removing water from the reaction mixture, and b) the resulting polymer is applied to the substrate to give an edible surface coating.

The polymers of the present invention have the advantage that they do not comprise any unknown molecules/compounds as this is the case in natural shellac.

Since the process according to the invention starts from aleuritic acid and not its derivates, e.g. methyl aleuriteate or isopropyl aleuriteate, the resulting polymer is free from by-products like methanol or isopropanol.

The properties of the polymers obtainable by the process of the present invention, especially melting temperature and glass transition temperature can be designed by process parameters or the use of additional monomers different from aleuritic acid.

The processes according to the invention are described by way of example hereinafter, without any intention that the invention be restricted to these illustrative embodiments. When ranges, general formulae or classes of compounds are specified below, these are intended to encompass not only the corresponding ranges or groups of compounds which are explicitly mentioned but also all subranges and subgroups of compounds which can be obtained by leaving out individual values (ranges) or compounds. Where documents are cited in the context of the present description, their content shall fully form part of the disclosure content of the present invention, particularly in respect of the matters referred to. Percentages specified hereinbelow are by weight unless otherwise stated. Where average values are reported hereinafter, these are the numerical average, unless stated otherwise. Where properties of a material are referred to hereinafter, for example viscosities or the like, these are the properties of the material at 25°C, unless stated otherwise. Where chemical (empirical) formulae are used in the present invention, the specified indices may be not only absolute numbers but also average values.

The process according for preparing a substrate comprising an edible surface coating, is characterized in that a) a polymer is prepared by reacting at least aleuritic acid as monomer at a temperature of above 100 °C, preferably of from 120 °C to 150 °C while removing water from the reaction mixture, and b) the resulting polymer is applied to the substrate to give an edible (suitable for consumption) surface coating.

Depending on the composition of the polymer to be prepared further monomers as mentioned below might be added to the reaction mixture. The further monomers might be added to the reaction mixture at the same time as the aleuritic acid or before or after the addition of the aleuritic acid. Preferably the addition of any further monomers to the reaction mixture is done at the same time as the addition of the aleuritic acid.

Preferably the acid value of the reaction mixture is determined regularly (as described below) and the reaction of step a) is terminated when an acid value below 60, more preferably an acid value of from 17.5 to below 50 is reached.

The process is preferably conducted as a melt condensation. For this purpose, the monomers are preferably initially charged and melted. The polycondensation preferably takes place in the mel,t preferably at temperatures of from 120 to 150°C, preferably in the course of 2 to 100 hours, preferably 5 to 75 hours.

Preferably the process of the invention is conducted in the absence of oxygen. More preferably the process of the invention if conducted under inert gas atmosphere, preferably using nitrogen as inert gas.

It may be advantageous if a majority of the amount of water released is initially distilled off at standard pressure. In the further course, the remaining water of reaction are preferably eliminated by distillation, until the target acid number is achieved. Optionally this may be made easier through reduced pressure, through an enlargement in the surface area, or by the passing of an inert gas stream through the reaction mixture. A preferred inert gas is nitrogen.

It may be advantageous to add further additives and processing auxiliaries, such as antioxidants or color stabilizers, to the reaction (esterification) mixture.

In one preferred embodiment of the process of the invention the polymer is prepared using only aleuritic acid as monomer.

In a more preferred embodiment of the process according to the invention the polymer is prepared using further monomer(s), preferably one or more hydroxy groups comprising organic acids, preferably mandelic acid, lactic, hydroxy stearic acid and/or glycolic acid, and/or dicarboxylic acid, preferably succinic acid, and/or dihydroxy-compounds, preferably difunctional di-propylene glycol mono methyl ether as monomer(s). More preferably the polymer is prepared using up to 30 mol-%, more preferably of from 10 to 30 mol-% based on the total of monomers used, of one or more hydroxy groups comprising organic acids, preferably mandelic acid, lactic, hydroxy stearic acid and/or glycolic acid, and/or dicarboxylic acid, preferably succinic acid, and/or dihydroxy-compounds, preferably a dihydroxy-functional di-propylene glycol mono methyl ether (DPGME) available from Perstorp Holding AB under the tradename YMER^{™} N120 as monomer(s) preferably on polyethylene glycol or its derivates as monomers.

It might be advantageous to use only one further monomer, preferably hydroxy stearic acid, lactic acid or mandelic acid. Those further monomers are preferably used in an amount of up to 30 mol-%, more preferably of from 10 to 30 mol-% based on the total of monomers used.

It might also be advantageous to use at least two, preferably exactly two further monomers, preferably lactic acid and mandelic acid, or succinic acid and DPGME. The amount of the two further monomers preferably adds up to 30 mol-%, more preferably of from 10 to 30 mol-% based on the total of monomers used.

Preferably the polymer obtained by the process according to the invention comprises at least 70 mol-% of units that are based on aleuritic acid as monomer.

In a preferred process according to the invention the polymer has a number averaged molecular weight Mn, determined by the method given below, of from 300 to 6000 g/mol.

It might be advantageous when the polymer has a weight averaged molecular weight Mw, determined by the method given in the description, of from 2500 to 175000, preferably of from 12500 to 150000 g/mol.

The polymer preferably has a OH-value, determined by the method given below, of from 200 to 400.

Preferably the polymer (obtained in step a) has a glass transition temperature Tg, determined by the method given in the description, of from -10 to -60 °C more preferably of from -10 to -30 °C.

The polymer produced by step a) of the process according to the invention preferably comprises at least 70 mol-% of the units, more preferably 70 to 100 mol% of the units are based on aleuritic acid as monomer and the polymer preferably has a weight averaged molecular weight Mw, determined by the method given in the description, of from 2500 to 175000 g/mol, preferably of from 12500 to 150000 g/mol.

The most preferred polymer obtained by step a) of the process according to the invention has a number averaged molecular weight of from 300 to 6000 g/mol, a weight averaged molecular weight of from 15000 to 150000 g/mol, an OH-value of from 200 to 400, and a glass transition temperature Tg of from -10 to -60 °C, preferably of from -10 to -30°C.

The units that are based on one or more hydroxy groups comprising organic acids, di-carboxylic acids or dihydroxy-compounds might be distributed in the polymer randomly or as one or more oligomeric blocks.

Preferably the polymer prepared according to the invention comprises up to 50 mol-%, more preferably of from 1 to 40 mol-%, and most preferably of from 10 to 30 mol-% of units that are based on lactic acid and/or mandelic acid as monomer. The use of these polymers results in highly transparent, and hard but flexible films.

In another preferred embodiment of the invention the polymer prepared comprises up to 50 mol-%, more preferably of from 1 to 40 mol-%, and most preferably of from 10 to 30 mol-% of units that are based on succinic caid and/or difunctional di-propylene glycol mono methyl ether as monomer. These polymers are slightly softer than the other polymers.

In a further preferred embodiment of the invention the polymer prepared comprises up to 50 mol-%, more preferably of from 1 to 40 mol-%, and most preferably of from 10 to 30 mol-% of units that are based on hydroxy stearic acid as monomer. The use of these polymers results in films much more hydrophobic than the other polymers.

It might be advantageous if the polymer used according to the invention consists only of units based on aleuritic acid as monomer.

The water vapor permeability of the polymers/copolymers prepared can be designed by varying the amount of comonomer used to prepare the copolymer (the amount of comonomer present in the polymer prepared).

The composition of the polymer can be determined by NMR-spectroscopy as described hereinafter. 500 mg of the polymer are dissolved in 0.5 mL d-DSMO (Hexadeuterodimethyl sulfoxide). ¹³C-NMR spectra and ¹H-NMR spectra were prepared using a Bruker Avance III HD spectrometer, (1H 500.1 MHz = 11,7 Tesla)
Probehead: 5mm Prodigy Cryo-Probehead (CPPBO)
Experiments: ¹H, ¹³C, ¹³C DEPT135
Number of scans: 32 (¹H), 2048 (¹³C).

The substrate used in step b) might for example be selected from pharmaceutical goods, more preferably from oral applicable tablets, pills, or capsules.

Step b) of the process according to the invention might be performed in any way known in the art. Preferably polymer is applied in an amount of from 1 to 15 mg/cm², preferably 2 to 8 mg/cm² to the surface of the substrate.

Preferably the polymer is applied as a solution (molecular dispersion) of the polymer or as a dispersion of the polymer. Preferably the solution or dispersion comprises of from 1 to 50 % by weight, preferably of from 2 to 25 % by weight and more preferably 5 to 20 % by weight of the polymer obtained in step a) based on the total weight of the dispersion/solution.

The polymer obtained in step a) can be solved / dispersed in any known solvents / dispersing medium. However, nonpolar solvent / dispersing media are more suitable compared to polar solvents / dispersing media. Preferred solvents / dispersion medias are 1-butanol, ethyl acetate, i-propanol, ethanol, mixtures of ethanol and water, preferably comprising of from 40 vol-% to 70 vol% of ethanol, water, 0.1N NH₄, or 0.1 N NaOH. Most preferred solvent / dispersion medium is 1-butanol.

The present invention will be further illustrated by the following nonlimiting examples which indicate further features, embodiments, aspects and advantages of the present invention.

### Examples:

### 1. Test methods:

### a) Determination of acid value (AV):

The concentration of acid end groups is determined in accordance with ASTM D4662 and ASTM D7253 by titrimetric means using a Solvotrode easyClean (6.0229.020) from Metrohm as electrode as described in more detail the Metrohm Application Bulletin 200/3 e dated May 2019. The AV is given in mg KOH/g of polymer.

### b) Determination of OH value (OHV):

The concentration of the OH groups is determined in accordance with ASTM E1899-16 by titrimetric means using a Solvotrode easyClean (6.0229.020) from Metrohm as electrode as described in more detail the Metrohm Application Bulletin 200/3 e dated May 2019, and using dimethylformamide (DMF) instead of acetonitrile. The OHV is given in mg KOH/g of polymer.

### c) Determination of melting temperature Tm₁ and Tm₂:

The thermal properties of the polyesters used in the context of the present invention are determined by differential scanning calorimetry (DSC) in accordance with the DSC method DIN 53765. The values of the second heating interval are stated, and the heating rate was 10 K/min.

### d) Determination of viscosity:

The viscosity of the polyesters produced and of the reaction products of polyester and diisocyanate was determined in accordance with DIN EN ISO 3219 in Pa.s using a rotational viscometer at the temperature specified in each case.

### e) Determination of glass transition temperature Tg:

The thermal properties of the polyesters used in the context of the present invention are determined by differential scanning calorimetry (DSC) in accordance with the DSC method DIN 53765. The values of the second heating interval are stated, and the heating rate was 10 K/min.

### f) Determination of molecular weight:

The number-average molecular weight and the weight-average molecular weight of the polyesters according to the invention is determined in accordance with DIN 55672-1 by means of gel permeation chromatography in tetrahydrofuran as eluent and polystyrene for calibration.

### 2. Raw Materials used

**Table 1: Raw materials used, trade names and producer**

| Chemical name | Abbreviation | Trade name | Producer |
|---|---|---|---|
| Aleuritic acid | / | DL-erythro-Aleuritic acid | Taros Chemicals GmbH & Co. KG |
| OHV: 563 mg KOH/g | | | |
| Aleuritic acid | / | Aleuritic acid | Dalian Stars Trading Co., Ltd. |
| OHV: 483 mg KOH/g | | | |
| Aleuritic acid | / | Aleuritic acid | Dalian Stars Trading Co., Ltd. |
| OHV: 454 mg KOH/g | | | |
| Aleuritic acid | / | Aleuritic acid | Dalian Stars Trading Co., Ltd. |
| OHV: 433 mg KOH/g | | | |
| Aleuritic acid | / | Aleuritic acid | Foreverest Resources LTD. |
| OHV: 529 mg KOH/g | | | |
| Aleuritic acid | / | Aleuritic acid | Dalian Stars Trading Co., Ltd. |
| OHV: 449 mg KOH/g | | | |
| Aleuritic acid | / | Aleuritic acid | Foreverest Resources LTD. |
| OHV: 520 mg KOH/g | | | |
| Aleuritic acid | / | Aleuritic acid | Beckmann-Kenko GmbH |
| OHV: 509 mg KOH/g | | | |
| Succinic acid | | Succinic acid | Th. Geyer GmbH & Co. KG |
| Linear dihydroxy-functional polyethylene glycol monomethyl ether OHV: 100 to 120 mg KOH/g | DPGME | Ymer^{™} N120 | Perstorp Holding AB |
| 12-hydroxy stearic acid | | 12-Hydroxy Stearinsäure | PENPET Petrochemical Trading GmbH |
| Glycolic acid | / | Glycolic acid, 98%, trading unit: 500 GRM, CAS-No: 79-14 | Th. Geyer GmbH & Co. KG |
| Aqueous L-lactic acid (80 % by weight) | LA80 | CAS-No: 79-33-4 | Carl Roth GmbH & Co KG |
| DL-Mandelic acid (for synthesis) | | | Sigma Aldrich |

### 3. Synthesis of polyesters

### a.) Aleuritic acid homo-polymer

Aleuritic acid OHV 563 mg KOH/g (60.0 g, 0.20 mol) were filled under a nitrogen stream into a 100 mL reaction flask, comprising a temperature probe, a distillation bridge, and a stirrer and heated. Within 90 minutes the temperature was continuously raised to 180 °C while stirring. After 2 hours at that temperature the viscosity of the melt increased spontaneously, and the product became a gel. The characteristics of the product could not be determined due to the insolubility of the product.

### b.) Aleuritic acid copolyester

Aleuritic acid OHV: 563 mg KOH/g (104.5 g, 0.34 mol), succinic acid (5.1 g 0.04 mol) and DPGME (45.0 g, 0.04 mol) were filled under a nitrogen stream into a 250 mL reaction flask, comprising a temperature probe, a distillation bridge, and a stirrer and heated. Within 45 minutes the temperature was raised continuously to 130 °C while stirring. After 31 hours at that temperature an acid number of 25.4 was reached and the reaction was stopped by terminating the heating and the reaction mixture was cooled down to room temperature. The characteristics of the product obtained are given in table 2 below.

### c.) Aleuritic acid homo-polymer

Aleuritic acid OHV 563 mg KOH/g (60.0 g, 0.20 mol) were filled under a nitrogen stream into a 250 mL reaction flask, comprising a temperature probe, a distillation bridge, and a stirrer and heated. Within 45 minutes the temperature was raised continuously to 130 °C while stirring. After 34 hours at that temperature an acid number of 21.7 was reached and the reaction was stopped by terminating the heating and the reaction mixture was cooled down to room temperature. The characteristics of the product obtained are given in table 2 below.

### d.) Aleuritic acid copolyester

Aleuritic acid OHV: 563 mg KOH/g (104.5 g, 0.34 mol), succinic acid (5.1 g 0.04 mol) and DPGME (45.0 g, 0.04 mol) were filled under a nitrogen stream into a 250 mL reaction flask, comprising a temperature probe, a distillation bridge, and a stirrer and heated. Within 55 minutes the temperature was raised continuously to 130 °C while stirring. After 46 hours at that temperature an acid number of 25.0 was reached and the reaction was stopped by terminating the heating and the reaction mixture was cooled down to room temperature. The characteristics of the product obtained are given in table 2 below.

### e.) Aleuritic acid copolyester

Aleuritic acid OHV: 563 mg KOH/g (85.0 g, 0.28 mol), succinic acid (7.1 g 0.06 mol) and DPGME (62.7 g, 0.06 mol) were filled under a nitrogen stream into a 250 mL reaction flask, comprising a temperature probe, a distillation bridge, and a stirrer and heated. Within 50 minutes the temperature was raised continuously to 130 °C while stirring. After 46 hours at that temperature an acid number of 24.6 was reached and the reaction was stopped by terminating the heating and the reaction mixture was cooled down to room temperature. The characteristics of the product obtained are given in table 2 below.

### f.) Aleuritic acid copolyester

Aleuritic acid OHV: 563 mg KOH/g (77.8 g, 0.26 mol) and hydroxy stearic acid (76.8 g, 0.26 mol) were filled under a nitrogen stream into a 250 mL reaction flask, comprising a temperature probe, a distillation bridge, and a stirrer and heated. Within 30 minutes the temperature was raised continuously to 130 °C while stirring. After 65 hours at that temperature an acid number of 18.9 was reached and the reaction was stopped by terminating the heating and the reaction mixture was cooled down to room temperature. The characteristics of the product obtained are given in table 2 below.

### g.) Aleuritic acid copolyester

Aleuritic acid OHV: 563 mg KOH/g (108.6 g, 0.36 mol) and hydroxy stearic acid (46.0 g, 0.15 mol) were filled under a nitrogen stream into a 250 mL reaction flask, comprising a temperature probe, a distillation bridge, and a stirrer and heated. Within 30 minutes the temperature was raised continuously to 130 °C while stirring. After 41 hours at that temperature an acid number of 21.9 was reached and the reaction was stopped by terminating the heating and the reaction mixture was cooled down to room temperature. The characteristics of the product obtained are given in table 2 below.

### h.) Aleuritic acid copolyester

Aleuritic acid OHV: 563 mg KOH/g (139.2 g, 0.46 mol) and hydroxy stearic acid (15.2 g, 0.05 mol) were filled under a nitrogen stream into a 250 mL reaction flask, comprising a temperature probe, a distillation bridge, and a stirrer and heated. Within 30 minutes the temperature was raised continuously to 130 °C while stirring. After 35 hours at that temperature an acid number of 28.5 was reached and the reaction was stopped by terminating the heating and the reaction mixture was cooled down to room temperature. The characteristics of the product obtained are given in table 2 below.

### i) Aleuritic acid homo-polymer

Aleuritic acid OHV 483 mg KOH/g (180.0 g, 0.59 mol) were filled under a nitrogen stream into a 250 mL reaction flask, comprising a temperature probe, a distillation bridge, and a stirrer and heated. Within 45 minutes the temperature was raised continuously to 130 °C while stirring. After 9 hours at that temperature the viscosity of the melt increased spontaneously, and the product became a gel. The characteristics of the product could not be determined due to the insolubility of the product.

### j.) Aleuritic acid homo-polymer

Aleuritic acid OHV 483 mg KOH/g (150.0 g, 0.49 mol) were filled under a nitrogen stream into a 250 mL reaction flask, comprising a temperature probe, a distillation bridge, and a stirrer and heated. Within 45 minutes the temperature was raised continuously to 130 °C while stirring. After 8 hours at that temperature an acid number of 56.0 was reached and the viscosity increased spontaneously. Measurable characteristics of the product obtained are given in table 2 below.

### k.) Aleuritic acid homo-polymer

Aleuritic acid OHV 454 mg KOH/g (150.0 g, 0.49 mol) were filled under a nitrogen stream into a 250 mL reaction flask, comprising a temperature probe, a distillation bridge, and a stirrer and heated. Within 30 minutes the temperature was raised continuously to 130 °C while stirring. After 17 hours at that temperature the viscosity of the melt increased spontaneously, and the product became a gel. The characteristics of the product could not be determined due to the insolubility of the product. The last acid number determined (after about 15 hours) was 39.4.

### l.) Aleuritic acid homo-polymer

Aleuritic acid OHV 433 mg KOH/g (150.0 g, 0.49 mol) were filled under a nitrogen stream into a 250 mL reaction flask, comprising a temperature probe, a distillation bridge, and a stirrer and heated. Within 60 minutes the temperature was raised continuously to 130 °C while stirring. After 10 hours at that temperature an acid number of 38.2 was reached and the reaction was stopped by terminating the heating and the reaction mixture was cooled down to room temperature. The characteristics of the product obtained are given in table 2 below.

### m.) Aleuritic acid homo-polymer

Aleuritic acid OHV 454 mg KOH/g (150.0 g, 0.49 mol) were filled under a nitrogen stream into a 250 mL reaction flask, comprising a temperature probe, a distillation bridge, and a stirrer and heated. Within 40 minutes the temperature was raised continuously to 130 °C while stirring. After 9 hours at that temperature an acid number of 42.1 was reached and the reaction was stopped by terminating the heating and the reaction mixture was cooled down to room temperature. The characteristics of the product obtained are given in table 2 below.

### n.) Aleuritic acid homo-polymer

Aleuritic acid OHV 454 mg KOH/g (1200.0 g, 3.90 mol) were filled under a nitrogen stream into a 2000 mL reaction flask, comprising a temperature probe, a dephlegmator, a distillation bridge, and a stirrer and heated. Within 50 minutes the temperature was raised continuously to 130 °C while stirring. After 17 hours at that temperature an acid number of 43.3 was reached and the reaction was stopped by terminating the heating and the reaction mixture was cooled down to room temperature. The characteristics of the product obtained are given in table 2 below.

### o.) Aleuritic acid homo-polymer

Aleuritic acid OHV 454 mg KOH/g (1200.0 g, 3.90 mol) were filled under a nitrogen stream into a 2000 mL reaction flask, comprising a temperature probe, a dephlegmator, a distillation bridge, and a stirrer and heated. Within 50 minutes the temperature was raised continuously to 130 °C while stirring. After 15 hours at that temperature an acid number of 43.1 was reached and the reaction was stopped by terminating the heating and the reaction mixture was cooled down to room temperature. The characteristics of the product obtained are given in table 2 below.

### p.) Aleuritic acid homo-polymer

Aleuritic acid OHV 454 mg KOH/g (1200.0 g, 3.90 mol) were filled under a nitrogen stream into a 2000 mL reaction flask, comprising a temperature probe, a dephlegmator, a distillation bridge, and a stirrer and heated. Within 60 minutes the temperature was raised continuously to 130 °C while stirring. After 16 hours at that temperature an acid number of 42.4 was reached and the reaction was stopped by terminating the heating and the reaction mixture was cooled down to room temperature. The characteristics of the product obtained are given in table 2 below.

### q.) Aleuritic acid homo-polymer

Aleuritic acid OHV 529 mg KOH/g (150.0 g, 0.49 mol) were filled under a nitrogen stream into a 250 mL reaction flask, comprising a temperature probe, a distillation bridge, and a stirrer and heated. Within 30 minutes the temperature was raised continuously to 130 °C while stirring. After 25 hours at that temperature an acid number of 26.1 was reached and the reaction was stopped by terminating the heating and the reaction mixture was cooled down to room temperature. The characteristics of the product obtained are given in table 2 below.

### r.) Aleuritic acid homo-polymer

Aleuritic acid OHV 449 mg KOH/g (1200.0 g, 3.90 mol) were filled under a nitrogen stream into a 2000 mL reaction flask, comprising a temperature probe, a dephlegmator, a distillation bridge, and a stirrer and heated. Within 60 minutes the temperature was raised continuously to 130 °C while stirring. After 11 hours at that temperature an acid number of 41.8 was reached and the reaction was stopped by terminating the heating and the reaction mixture was cooled down to room temperature. The characteristics of the product obtained are given in table 2 below.

### s.) Aleuritic acid homo-polymer

Aleuritic acid OHV 529 mg KOH/g (1200.0 g, 3.90 mol) were filled under a nitrogen stream into a 2000 mL reaction flask, comprising a temperature probe, a dephlegmator, a distillation bridge, and a stirrer and heated. Within 30 minutes the temperature was raised continuously to 130 °C while stirring. After 20 hours at that temperature an acid number of 26.6 was reached and the reaction was stopped by terminating the heating and the reaction mixture was cooled down to room temperature. The characteristics of the product obtained are given in table 2 below.

### t.) Aleuritic acid homo-polymer

Aleuritic acid OHV 520 mg KOH/g (1200.0 g, 3.90 mol) were filled under a nitrogen stream into a 2000 mL reaction flask, comprising a temperature probe, a dephlegmator, a distillation bridge, and a stirrer and heated. Within 60 minutes the temperature was raised continuously to 130 °C while stirring. After 32 hours at that temperature an acid number of 27.0 was reached and the reaction was stopped by terminating the heating and the reaction mixture was cooled down to room temperature. The characteristics of the product obtained are given in table 2 below.

### u.) Aleuritic acid homo-polymer

Aleuritic acid OHV 520 mg KOH/g (1200.0 g, 3.90 mol) were filled under a nitrogen stream into a 2000 mL reaction flask, comprising a temperature probe, a dephlegmator, a distillation bridge, and a stirrer and heated. Within 30 minutes the temperature was raised continuously to 130 °C while stirring. After 35 hours at that temperature an acid number of 26.7 was reached and the reaction was stopped by terminating the heating and the reaction mixture was cooled down to room temperature. The characteristics of the product obtained are given in table 2 below.

### v.) Aleuritic acid copolyester

Aleuritic acid, AV: 175.5 mg KOH/g (1002.7 g, 3.29 mol) and mandelic acid (65.03 g, 0.43 mol) were filled under a nitrogen stream into a 2 L reaction flask, comprising a temperature probe, a distillation bridge, and a stirrer and heated. Within 30 minutes the temperature was raised continuously to 140 °C while stirring. After 24 hours at that temperature an acid number of 38.0 was reached and the reaction was stopped by terminating the heating and the reaction mixture was poured on a baking plate and cooled using dry ice. The characteristics of the product obtained are given in table 2 below.

### w.) Aleuritic acid copolyester

Aleuritic acid, AV: 175.5 mg KOH/g (900 g, 2.96 mol) and mandelic acid (135 g, 0.89 mol) were filled under a nitrogen stream into a 2 L reaction flask, comprising a temperature probe, a distillation bridge, and a stirrer and heated. Within 30 minutes the temperature was raised continuously to 140 °C while stirring. After 24 hours at that temperature an acid number of 35.0 was reached and the reaction was stopped by terminating the heating and the reaction mixture was poured on a baking plate and cooled using dry ice. The characteristics of the product obtained are given in table 2 below.

### x.) Aleuritic acid copolyester

Aleuritic acid, AV: 175.5 mg KOH/g (1000 g, 3.29 mol) and LA80 (48.1 g, 0.43 mol) were filled under a nitrogen stream into a 2 L reaction flask, comprising a temperature probe, a distillation bridge, and a stirrer and heated. Within 30 minutes the temperature was raised continuously to 140 °C while stirring. After 24 hours at that temperature an acid number of 39.0 was reached and the reaction was stopped by terminating the heating and the reaction mixture was poured on a baking plate and cooled using dry ice. The characteristics of the product obtained are given in table 2 below.

### y.) Aleuritic acid copolyester

Aleuritic acid, AV: 175.5 mg KOH/g (1001.6 g, 3.29 mol) and LA80 (110.96 g, 0.99 mol were filled under a nitrogen stream into a 2 L reaction flask, comprising a temperature probe, a distillation bridge, and a stirrer and heated. Within 30 minutes the temperature was raised continuously to 140 °C while stirring. After 24 hours at that temperature an acid number of 50.5 was reached and the reaction was stopped by terminating the heating and the reaction mixture was poured on a baking plate and cooled using dry ice. The characteristics of the product obtained are given in table 2 below.

### z.) Aleuritic acid copolyester

Aleuritic acid AV: 175.5 mg KOH/g (900.3 g, 2.96 mol), LA80 (33.3 g, 0.3 mol), and mandelic acid (89.9 g, 0.59 mol) were filled under a nitrogen stream into a 2 L reaction flask, comprising a temperature probe, a distillation bridge, and a stirrer and heated. Within 30 minutes the temperature was raised continuously to 140 °C while stirring. After 24 hours at that temperature an acid number of 37.0 was reached and the reaction was stopped by terminating the heating and the reaction mixture was poured on a baking plate and cooled using dry ice. The characteristics of the product obtained are given in table 2 below.

**Table 2: Characteristics of the polymers 3a.) to 3z.)**

| Example | Mn [g/mol] | Mw [g/mol] | Tg [°C] | Tm₁ [°C] | Tm₂ [°C] | OHV [mg KOH/g] | AV [mg KOH/g] |
|---|---|---|---|---|---|---|---|
| 3 a.) | n.d. | n.d. | n.d. | n.d. | n.d. | n.d. | n.d. |
| 3 b.) | 3900 | 25800 | -57.3 | 28.9 | 37.6 | 260 | 25.4 |
| 3 c.) | 5600 | 66400 | -11.8 | 51.9 | - | 368 | 21.7 |
| 3 d.) | 3900 | 28500 | -56.8 | 25.0 | 38.8 | 262 | 25.0 |
| 3 e.) | 3700 | 19300 | -58.1 | 25.7 | - | 220 | 24.6 |
| 3 f.) | 5700 | 132000 | -26.7 | 14.9 | - | - | 18.9 |
| 3 g.) | 5100 | 80300 | -22.1 | 34.4 | - | 241 | 21.9 |
| 3 h.) | 4800 | 41700 | -14.9 | 46.9 | - | 340 | 28.5 |
| 3 i.) | n.d. | n.d. | n.d. | n.d. | n.d. | n.d. | n.d. |
| 3 j.) | n.d. | n.d. | n.d. | n.d. | n.d. | 328 | 56.0 |
| 3 k.) | n.d. | n.d. | n.d. | n.d. | n.d. | n.d. | n.d. |
| 3 l.) | 3800 | 147000 | -15.7 | 45.8 | 65.8 | 314 | 38.2 |
| 3 m.) | 3200 | 19500 | -14.0 | 45.4 | - | 330 | 42.1 |
| 3 n.) | 3200 | 19400 | -14.6 | 44.9 | - | 340 | 41.7 |
| 3 o.) | 3100 | 17200 | -15.1 | 44.6 | - | 340 | 43.1 |
| 3 p.) | 3300 | 21200 | -13.7 | 45.6 | - | 345 | 42.4 |
| 3 q.) | 4500 | 54400 | -11.2 | 50.8 | - | 375 | 26.1 |
| 3 r.) | 3400 | 37500 | -16.2 | 45.5 | 55.8 | 322 | 41.8 |
| 3 s.) | 4400 | 41300 | -12.1 | 49.9 | - | 364 | 26.6 |
| 3 t.) | 4400 | 36500 | -11.2 | 51.3 | - | 378 | 27.0 |
| 3 u.) | 4400 | 37900 | -11.1 | 51.0 | - | 386 | 26.7 |
| 3 v.) | 1860 | 12200 | -13 | 52 | | n.d. | 38.0 |
| 3 w.) | 2270 | 20300 | -12 | 48 | | n.d. | 35.0 |
| 3 x.) | 1730 | 9450 | -15 | 54 | | n.d. | 39.0 |
| 3 y.) | 1500 | 6550 | -17 | 50 | | n.d. | 50.5 |
| 3 z.) | 2190 | 18600 | -13 | 49.0 | | n.d. | 37.0 |

| | | | | | | | |
|---|---|---|---|---|---|---|---|
| n.d.: not determined | | | | | | | |

### 4. Synthesis of aleuritic acid / glycolic acid co-polyester

### a.) Preparation of oligo glycolic acid

Glycolic acid (160.3 g, 2.11 mol) were filled in a nitrogen stream into a 250 mL reaction flask, comprising a temperature probe, a distillation bridge, and a stirrer and heated. Within 120 minutes the temperature was raised continuously to 150 °C while stirring. After 5 hours at that temperature the clear melt became a little bit turbid. The reaction was stopped by ending further heating and the melt obtained was filled into a storage flask. The characteristics of the product obtained are given in table 3 below.

### b.) Preparation of aleuritic acid / glycolic acid co-polyester

Oligo glykol acid from example 4 a.) and aleuritic acid OHV 509 mg KOH/g were filled under a nitrogen stream into a 1000 mL reaction flask, comprising a temperature probe, a distillation bridge, and a stirrer and heated. Within 30 minutes the temperature was raised continuously to 150 °C while stirring. After 10 hours at that temperature an acid number of 31.1 mg KOH/g was reached, and the reaction was stopped by terminating the heating and the reaction mixture was cooled down to room temperature. The characteristics of the product obtained are given in table 3 below.

**Table 3: Characteristics of the products of examples 4a.) and 4.b)**

| Example | Mn [g/mol] | Mw [g/mol] | Tg [°C] | Tm₁ [°C] | Tm₂ [°C] | OHV [mg KOH/g] | AV [mg KOH/g] |
|---|---|---|---|---|---|---|---|
| 4 a.) | - | - | - | - | - | - | 156 |
| 4 b.) | 4500 | - | -15.6 | 45.0 | - | 375 | 31.1 |

### 5. Coating of tablets and capsules

### a.) Preparing of film formulation

A 10 % by weight solution of the polymer in 1-butanol was prepared by adding 50 g of the polymer of example 3s.) to 450 g of 1-butanol (from Merck) and stirring at room temperature overnight. A colloidal solution was obtained.

### b.) Casted films

To test the film forming properties casted film tests were done as pretest. The 10 % by weight solution of the polymer in 1-butanol of example 5a was poured on a glass plate and dried for 24 hours at 40°C in circulating air oven. The film obtained was physically and optically tested on film formation, brittleness, stickiness, and glossiness. The polymer shows film forming properties, whereby the film was flexible, not sticky and glossy.

### c.) Tablet formulation

To further investigate the processability of the polymer a tablet coating was performed. A hydrophilic tablet core was produced to afterwards test the water protection capabilities of the film. All raw materials given in table 4 were weighed accurately. The raw materials were dried at 70°C for about 1 hour in a tray dryer for activation, afterwards the raw materials were mixed in a PE bag. The final blend was compressed using a 10 mm circular, standard-concave punch at a Korsch XP1 (13509 Berlin Germany).

**Table 4: Raw material composition of test tablet**

| Raw material | Supplier | Batch | Content [% by weight] |
|---|---|---|---|
| Silica Type 53-115µm | Sigma Aldrich | MKCD0974 | 15 |
| Silica Type 40-75µm | Sigma Aldrich | BCBH8948V | 2,5 |
| AEROSIL^{®} 200P | EVONIK | 159040414 | 1 |
| Avicel PH 102 | FMC Biopolymer | 71802C | 79,5 |
| AC-DI-SOL^{®} | Signet | T1642C | 1 |
| Sodium stearyl fumarate | ABCR Chemicals | AB222714 | 1 |
| Total | | | 100 |

### d.) Tablet coating process

To determine the processability 77.9 g of the polymer of example 3 s.) was dissolved in 1441.9 g 1-butanol (Merck) to obtain a solution to be used for tablet coating purposes. After the polymer was completely dissolved an amount of 39.0 g of talc (Talc M, Luzenac) was added and the mixture was stirred for additional 5 min. The coating suspension was stirred during the whole coating process.

The tablets produced in example 5c were used as substrate. The coating process was adjusted to gain coated tablets with about 8 mg/cm² of polymer applied to the tablet. As coating equipment an O'Hara Labcoat 1 (Ontario, Canada) was used with a batch size of 900 g. The process parameters used are given in table 5a and 5b. Samples were taken at 4, 6, and of course 8 mg/cm² of polymer applied.

**Table 5a: Process parameters**

| Operation | Time | Temperature | | | Inlet | Pump | |
|---|---|---|---|---|---|---|---|
| | | Inlet | Exhaust | Product | Air | Flow | |
| | | | | | vol. | rate | |
| | [min] | [°C] | [°C] | [°C] | [m³/h] | [g/min] | scale |
| Settings | | | | | | | |
| Spraying | 0 - 10 | 42.8 | 31.7 | 35.0 | 95 | 3.0 | 6 |
| Spraying | 10 - 30 | 42.0 | 33.7 | 35.9 | 120 | 4.0 | 8 |
| Spraying | 30 - 40 | 42.0 | 33.0 | 36.4 | 140 | 3.0 | 6 |
| Spraying | 40 - 60 | 40.2 | 34.2 | 35.4 | 140 | 3.0 | 6 |
| Spraying | 60 - 180 | 39.1 | 34.9 | 35.2 | 140 | 3.3 | 6.25 |
| Spraying | 180 - 470 | 39.0 | 35.4 | 36.4 | 140 | 3.3 | 6.25 |
| Drying | 0 - 10 | 31.2 | 32.3 | 31.9 | 140 | - | - |

**Table 5b: Process parameters (continue)**

| Operation | Time | Spray | | Pan | Diff. | Inlet | Room |
|---|---|---|---|---|---|---|---|
| | | press. | | speed | Press. | Temp. | Hum. |
| | | [bar] | | | | | |
| | [min] | atom. | flat | [rpm] | [cm H₂O] | [°C] | [rel. %] |
| Settings | | | | | | | |
| Spraying | 0 - 10 | 1.1 | 1.1 | 20 | -0.4 | 21.8 | 58.8 |
| Spraying | 10 - 30 | 1.1 | 1.1 | 20 | -0.4 | 22.0 | 57.1 |
| Spraying | 30 - 40 | 1.1 | 1.1 | 20 | -0.4 | 22.8 | 56.6 |
| Spraying | 40 - 60 | 1.5 | 1.5 | 18 | -0.4 | 22.9 | 56.5 |
| Spraying | 60 - 180 | 1.5 | 1.5 | 18 | -0.4 | 23.9 | 51.6 |
| Spraying | 180 - 470 | 1.5 | 1.5 | 18 | -0.4 | 24.5 | 51.4 |
| Drying | 0 - 10 | - | - | 3 | -0.4 | 24.5 | 51.3 |

### e.) Test of water uptake

The water uptake was tested using a super saturated aqueous KNO₃ solution. This solution was filled to the bottom of an exicator resulting in a relative humidity, which equals to 95% relative humidity at room temperature. The weight of the tablets was determined at the beginning and the increase in weight monitored after 1, 2, 3, 5, 7, and 9 days in the exicator by weighting the tablets again. The results are given in table 6.

**Table 6: water uptake of different tablets**

| Amount of polymer applied | 1 day | 2 days | 3 days | 5 days | 7 days | 9 days |
|---|---|---|---|---|---|---|
| [mg/cm²] | WI [%] | WI [%] | WI [%] | WI [%] | WI [%] | WI [%] |
| 4 | 5.07 | 8.05 | 9.28 | 12.43 | 13.23 | 13.85 |
| 6 | 2.36 | 4.57 | 7.31 | 11.42 | 12.69 | 13.39 |
| 8 | 0.65 | 1.30 | 1.91 | 5.26 | 8.03 | 10.32 |
| uncoated | 9.04 | 11.43 | 11.80 | 14.42 | 15.20 | 16.29 |

| | | | | | | |
|---|---|---|---|---|---|---|
| WI = weight increase | | | | | | |

It can be seen from table 6, that the water uptake can be reduced by coating of the tablet with the polymer according to the invention. The effect is larger in the first days and when using a higher amount of polymer coated to the surface of the test tablet.

### 6. Water vapor permeability

### a). Sample preparation

A 20 % by weight solutions of each of the polymers from example 3 v.) to 3 z.) in iso-propanol were prepared by adding 100 g of the polymers to 400 g of iso-propanol (from Merck) and stirring at 60 °C overnight. The solutions were coated using a doctor blade onto a water vapour permeable capacitor paper with a layer thickness of 150 µm. After drying over night at room temperature a capacitor paper comprising layer films of the polymers on top were obtained. Samples of 25 mm * 25 mm were cut-out from these capacitor papers.

A 30 ml wide-neck sample jar (opening diameter 22.5 mm) was filled with 5 g dry silica gel (Silica Gel Industry Grade CN 70.1, Carl Roth GmbH + Co. KG) and rubbed on the edge with stopcock grease (glisseal N, Borer Chemie AG). The cut-out paper samples were put onto the neck of the jar with the film layer side not in direction of the jar, the complete opening of the jar being closed by the paper sample. The paper samples were fixed by putting a screw female on top of the paper sample in a way, that the opening of the screw female is in alignment with the opening of the jar.

### b). Test procedure and results

The sample jars prepared in this way were placed in an desiccator filled with 150 mL saturated KCI solution. The mass increase was determined by the weight increase of the previously tara weighed sample jars. Three films of the polymers were always tested simultaneously, and a pure capacitor paper was tested as reference. Since the temperature and time were not constant in all tests, relative weight increase (water uptakes) were compared. The results are given in table 7.

**Table 7: water vapor permeability**

| Polymer sample applied | Weight increase in mg | Rel. weight increase in % |
|---|---|---|
| Blanko | 155.2 | 0 |
| 3 v.) | 32.33 | 20.8 |
| 3 x.) | 30.46 | 19.6 |
| Blanko | 153.2 | 0 |
| 3. w) | 59.63 | 38.93 |
| 3 y.) | 56.37 | 36.79 |
| Blanko | 141.3 | 0 |
| 3 z.) | 52.13 | 36.90 |

It can be seen from table 7, that the water uptake can be reduced by coating of the capacitor paper with the copolymer according to the invention. The water uptake can be regulated (controlled) by the aleuritic acid copolymer used. The water uptake can better be regulated (controlled) by using aleuritic acid copolymers with a higher or lower ratio (amount) of comonomer. The ability to uptake water can be increased by using copolymers with a higher amount of comonomer.

## Claims

1. Process for preparing a substrate comprising an edible surface coating, **characterized in that** a) a polymer is prepared by reacting at least aleuritic acid as monomer at a temperature of above 100 °C, preferably of from 120 °C to 150 °C while removing water from the reaction mixture, and b) the resulting polymer is applied to the substrate to give an edible surface coating.

2. Process according to claim 1, **characterized in that** the acid value of the reaction mixture is determined regularly and the reaction of step a) is terminated when an acid value below 60, more preferably of from 17.5 to below 50 is reached.

3. Process according to claim 1 or 2, **characterized in that** the polymer comprises at least 70 mol-% of units that are based on aleuritic acid as monomer.

4. Process according to any of claims 1 to 3, **characterized in that** the polymer has a number averaged molecular weight Mn, determined by the method given in the description, of from 300 to 6000 g/mol.

5. Process according to any of claims 1 to 4, **characterized in that** the polymer has a weight averaged molecular weight Mw, determined by the method given in the description, of from 2500 to 175000, preferably of from 12500 to 150000 g/mol.

6. Process according to any of claims 1 to 5, **characterized in that** the polymer has a OH-value, determined by the method given in the description, of from 200 to 400.

7. Process according to any of claims 1 to 6, **characterized in that** the polymer has a glass transition temperature Tg, determined by the method given in the description, of from -10 to -60 °C.

8. Process according to any of claims 1 to 7, **characterized in that** the polymer is prepared using only aleuritic acid as monomer.

9. Process according to any of claims 1 to 7, **characterized in that** the polymer is prepared in the presence of further monomer(s), preferably one or more hydroxy groups comprising organic acids, preferably mandelic acid, lactic, hydroxy stearic acid and/or glycolic acid, and/or dicarboxylic acid, preferably succinic acid, and/or dihydroxy-compounds, preferably difunctional di-propylene glycol mono methyl ether as monomer(s).

10. Process according to claim 8, **characterized in that** the polymer is prepared using up to 30 mol-%, more preferably of from 10 to 30 mol-% based on the total monomers used, of one or more hydroxy groups comprising organic acids, preferably mandelic acid, lactic, hydroxy stearic acid and/or glycolic acid, and/or dicarboxylic acid, preferably succinic acid, and/or dihydroxy-compounds, preferably dihydroxy-functional di-propylene glycol mono methyl ether as monomer(s).

11. Process according to any one of the preceding claims, **characterized in that** the substrate is selected from pharmaceutical goods, more preferably from oral applicable tablets, pills, or capsules.

12. Process according to any one or more of the preceding claims, **characterized in that** the polymer is applied in an amount of from 1 to 15 mg/cm², preferably 2 to 8 mg/cm² to the surface of the substrate.

13. Process according to any one or more of the preceding claims, **characterized in that** the polymer is applied as a solution of the polymer or as a dispersion of the polymer.
